# EUROPEAN PATENT APPLICATION

(11) **EP 2 330 419 A2**
(43) Date of publication of application: **08.06.2011**
(21) Application number: 09810129.8
(22) Date of filing: 09.07.2009
(51) Int. Cl.: G01N 33/50

(54) **NANO-SCALE BIOCHIP SCANNER**

(30) Priority: 25.08.2008 KR 20080082706
(71) Applicant: Korea Plating Materials Technology, Gyeonggi-do 425-090 (KR); Korea Electronics Technology Institute, Kyunggi-do 463-816 (KR)
(72) Inventor: HONG, Hyuck Ki, Yongin Gyeonggi-do 448-160 (KR); CHOI, Yeon Shik, Seoul 137-044 (KR); JO, Young Chang, Suwon Gyeonggi-do 443-470 (KR); SONG, Hyuck, Seongnam Gyeonggi-do 463-857 (KR)
(74) Representative: Fiesser, Gerold Michael
(86) International application number: PCT/KR2009/003748
(87) International publication number: WO 2010/024524

(57) **Abstract**

An apparatus for analyzing a biochip is provided. More particularly, the present invention provides a biochip scanner for emitting a line-type light with uniform intensity by alternately emitting the line-type light to both sides of a glass substrate holding a biochip labeled by a fluorescent material, and for analyzing a minimum bio sample by increasing the intensity of the fluorescence.
The biochip scanner includes a stage for holding a substrate on which a biochip is formed; a plurality of light output parts formed on the stage for outputting a line-type light to both sides of the substrate; and a camera disposed above the substrate.

## Description

### CROSS-REFERENCE TO RELATED APPLICATION(S) AND CLAIM OF PRIORITY

The present application claims the benefit under 35 U.S.C. § 119(a) to a Korean patent application filed in the Korean Intellectual Property Office on , and assigned Serial No. , the entire disclosure of which is hereby incorporated by reference.

### TECHNICAL FIELD OF THE INVENTION

The present invention relates generally to an apparatus for analyzing a biochip. More particularly, the present invention relates to a biochip scanner for emitting a line-type light with uniform intensity by alternately emitting the line-type light to both sides of a glass substrate holding a biochip labeled by a fluorescent material, and for analyzing a minimum bio sample by increasing the intensity of the fluorescence.

### BACKGROUND OF THE INVENTION

A biochip is a hybrid device formed in an existing semiconductor chip type by combining bio-organic matters isolated from creatures, such as enzymes, proteins, antibodies, DNA, microbes, animal and plant cells, organs and neurons, with inorganic matters such as semiconductors or glasses. The biochip acts to diagnose infectious diseases or analyze genes by using inherent functions of biomolecules and mimicking functions of organisms. The biochip acts as a novel function device for processing new information.

According to biomolecules and systemization, the biochip can be classified into a DNA chip, an RNA chip, a protein chip, a cell chip, and a neuron chip. Also, in a broad definition, the biochip can include a biosensor having detection and analysis functions of various biochemical materials, such as lab on a chip miniaturized and integrated and having automatic analysis functions including pretreatment of samples, biochemical reaction, detection, and data analysis.

A method for analyzing and inspecting the biochip forms a biochip array using a bio-reactant labeled with the fluorescent material, and then detects the emitted light of a particular wavelength by exciting the fluorescent material with the light of the particular wavelength. That is, when the fluorescent material receives the light of the particular wavelength, its internal energy increases and then decreases and emits the light of the wavelength longer than the excitation light.

In this case, a laser is mostly used as an external light source to excite the fluorescent material. The laser emits the light to the biochip and scans and detects a fluorescent signal from the biochip. When a plurality of fluorescent material types is used, their absorption wavelengths differ from each other. Thus, a plurality of excitation light sources is used.

A greater number of steps are required to fabricate and measure the biochip using the light as the excitation light. The fabricated biochip works by measuring the luminous wavelength and intensity from the external excitation light. Mostly, the biochip is fabricated using a glass substrate such as slide glass, and then its reactivity and level are observed using a scanner.

Meanwhile, the biochip is formed generally in an array structure. The reduction of the array size from the micro-size into the nanoscale size is quite important, which decrease the quantity and the volume of the used sample. Thus, the nanoscale biochip is advantageous and highly efficient in terms of the cost.

As the chip size reduces into the nanoscale range, the nanoarray enhances integration, sensitivity, and detection speed, compared to microarrays. The nanoarray allows high-density protein arrays to be used in biosensors or scanning technology of proteomics, and allows to research the analysis of the biomolecules in the molecular level.

However, in association with the researches and the applications relating to the nanoscale biochip, it is necessary to maintain biological activity and to effectively fix a sub-micron or nanoscale biomaterial to the biochip substrate. Also, it is required to develop techniques for effectively detecting characteristics of the nanoscale biochip by utilizing an appropriate analysis scheme on the sub-micron and nanoscale biochip array fabricated.

In the meantime, to pattern the biomaterial, various techniques are developed, such as microcontact printing, colloidal lithography, X-ray interference lithography, nanoprint lithography electron-beam lithography, focused ion beam, and Scanning Probe Lithography (SPL). Particularly, Dip-Pen Nano lithography (DPN) for forming a nano pattern on a surface using a tip of Atomic Force Microscope (AFM) is attracting attention as the representative of the SPL.

Main advantages in using the nanoscale biomaterial array forming technique with those schemes are to avoid non-specific binding of proteins or other biomolecules and to spatially control the fixation even in the small number of molecules, the single protein, or the DNA molecular level. Meanwhile, besides the generation of the nano patterns, the SPL additional reads the nano patterns but can restrictively obtain only structural (e.g., topography) characteristics.

However, when the nanoscale biomaterial array is formed, the chip scale is reduced. Thus, the intensity of the fluorescence decreases but the density of the biomaterial relatively increases. In result, it is difficult to analyze the biomaterial.

To address this shortcoming, a conventional method arranges a plurality of optical fibers in a row, converts the light coming from the light source to the line-type light, and then probes one side of the substrate.

However, the optical fiber, which includes a core layer for transferring the light and a clad layer surrounding the core layer, cannot emit the line-type light with the uniform intensity because the light intensity decreases in a region adjacent to the clad layer and the clad layer.

### SUMMARY OF THE INVENTION

To address the above-discussed deficiencies of the prior art, it is a primary aspect of the present invention to provide a biochip scanner for emitting a light of uniform intensity by converting a light from a light source to a line-type light using a geometric optics and including a plurality of light output parts in both sides of a glass substrate.

Another aspect of the present invention is to provide a biochip scanner for analyzing a biochip having a minimum bio sample by emitting a line-type light output from a light output part onto both sides of a substrate and supplying uniformly an energy required to excite a fluorescent material labeled in the biosample.

According to one aspect of the present invention, a biochip scanner includes a stage for holding a substrate on which a biochip is formed; a plurality of light output parts formed on the stage for outputting a line-type light to both sides of the substrate; and a camera disposed above the substrate.

The light output parts may be misaligned in both sides of the substrate.

The light output part may include a geometric optical system for producing a light emitted from a light source as a line light; an optical splitter and a plurality of mirrors for splitting the line light to a plurality of paths; and an light output part for emitting the line light split by the optical splitter, in both sides of the substrate.

The biochip scanner may further include a beam homogenizer disposed at an output stage of the light source, for making uniform intensity distribution of the light.

One end of the light output part formed in one side of the substrate may be formed in parallel with other end of the light output part formed in the other side of the substrate.

The light source may be a laser oscillator or a laser diode.

### BRIEF DESCRIPTION OF THE DRAWINGS

For a more complete understanding of the present disclosure and its advantages, reference is now made to the following description taken in conjunction with the accompanying drawings, in which like reference numerals represent like parts:

FIG. 1 is a diagram of a nanoscale biochip scanner according to an exemplary embodiment of the present invention;

FIG. 2 is a diagram of a light output part according to an exemplary embodiment of the present invention;

FIG. 3 is an intensity profile of a line-type light output from a plurality of light output parts according to an exemplary embodiment of the present invention;

FIG. 4 is an intensity profile of the light output from a light source including a beam homogenizer according to an exemplary embodiment of the present invention; and

FIG. 5 is a diagram of a nanoscale biochip scanner according to another exemplary embodiment of the present invention.

Throughout the drawings, like reference numerals will be understood to refer to like parts, components and structures.

### DETAILED DESCRIPTION OF THE INVENTION

Reference will now be made in detail to the embodiment of the present general inventive concept, examples of which are illustrated in the accompanying drawings, wherein like reference numerals refer to the like elements throughout. The embodiment is described below in order to explain the present general inventive concept by referring to the drawings.

The matters defined in the description such as a detailed construction and elements are provided to assist in a comprehensive understanding of the embodiments of the invention. Accordingly, those of ordinary skill in the art will recognize that various changes and modifications of the embodiments described herein can be made without departing from the scope and spirit of the invention.

Certain exemplary embodiments of the present disclosure will now be described in greater detail with reference to the accompanying drawings.

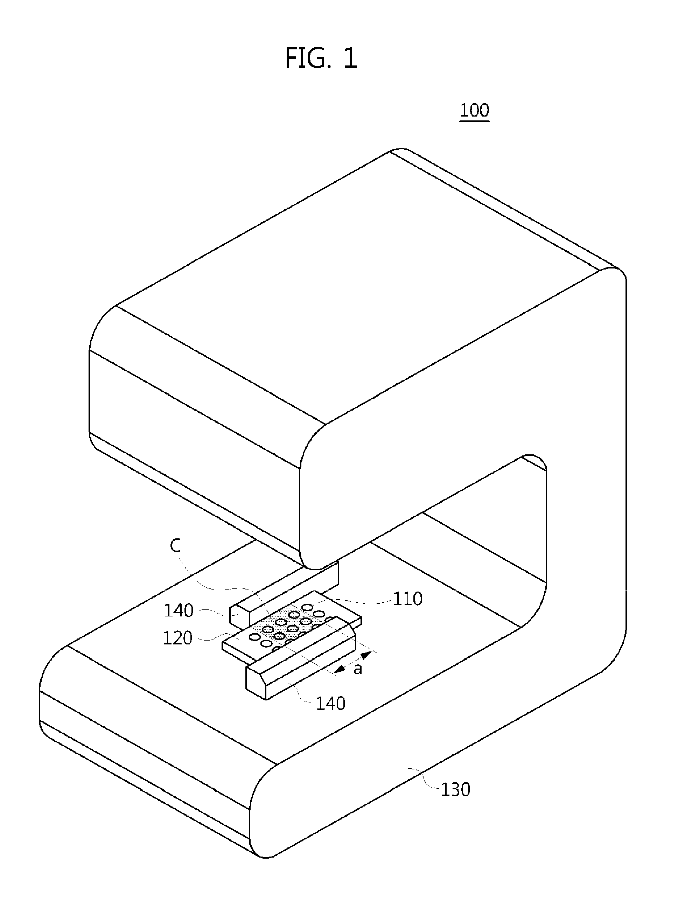

FIG. 1 is a diagram of a nanoscale biochip scanner according to an exemplary embodiment of the present invention.

The nanoscale biochip scanner 100 includes a stage 130 including a holder (not shown) for receiving a glass substrate 120 on which a biosample 110 is prepared, and a plurality of light output parts 140 for emitting line-type light to both sides of the glass substrate 120 fixed to the holder.

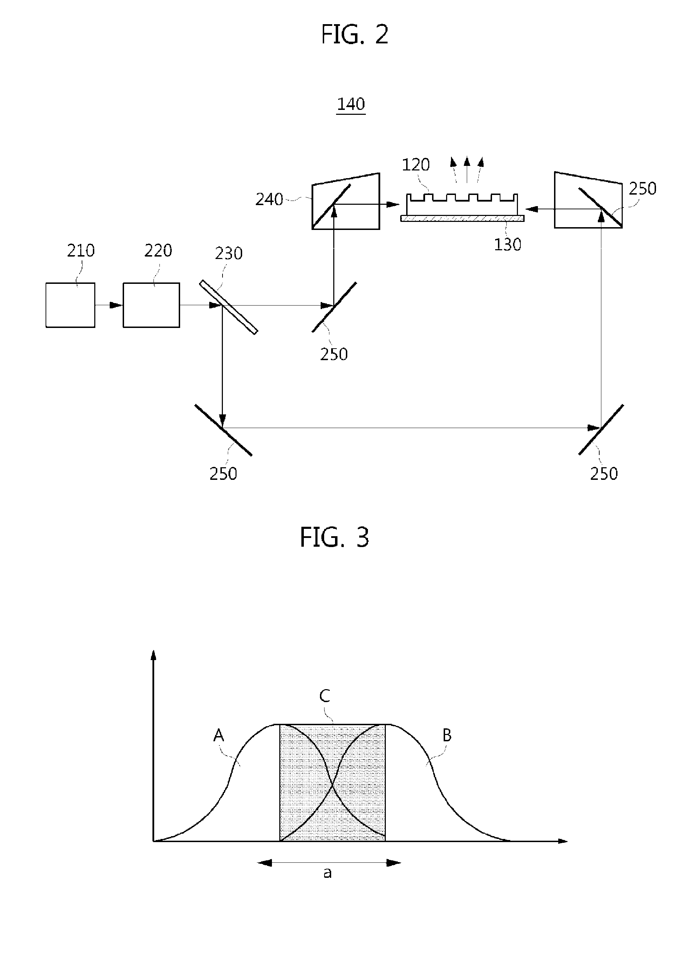

FIG. 2 is a diagram of the light output part according to an exemplary embodiment of the present invention.

The light output part 140 includes a light source 210 including a laser oscillator or a laser diode for outputting the light, a geometric optical system 220 for converting the light output from the light source 210 to a line-type light, an optical splitter 230 for splitting the line-type light into a plurality of paths, and a plurality of mirrors 250 for sending the line-type light split by the optical splitter 230 to the plurality of the lights, to a plurality of light output parts 240.

When the laser diode is used as the light source, volume and size of the present nanoscale biochip scanner are reduced and thus the nanoscale biochip scanner can be carried along.

The line-type light output from the light output part 240 is emitted to both sides of the glass substrate 120 placed on the stage 130, and the line-type light emits the light by exciting a fluorescent material to label the biosample 110.

The intensity of the line-type light output from the light output part 140 is in a form of Gaussian profile. When the line-type light having the Gaussian profile intensity is emitted to the side of the glass substrate 120, reliability of the test is degraded because the line-type light is emitted to each biosample with different intensities.

This problem can be addressed using a plurality of, for example, two light output parts 140. After the Gaussian profile of the line-type light output from each light output part 140 is analyzed, the light output parts 140 are positioned in both sides of the glass substrate 120 on a condition that the light output parts 140 are overlapped to generate a certain intensity; that is, on a condition that the centers of the light output parts 140 are spaced apart by a certain distance.

FIG. 3 depicts the intensity profile of the line-type light output from the plurality of the light output parts according to an exemplary embodiment of the present invention.

The intensity profile of the line-type light output from the light output part disposed in one side of the glass substrate is A, and the intensity profile of the line-type light output from another light output part disposed in the other side of the glass substrate is B. The length of the bottom side of each Gaussian profile can be understood as the length of the light output part or the length of the line-type light.

As shown in FIG. 3, when the distance between the centers of the light output parts is spaced by a, the line-type light output from each light output part forms a region having a constant light intensity C. The distance a in FIG. 1 is defined as such.

A CCD camera (not shown) for observing the fluorescence generated from the biosample 110 labeled with the fluorescent material by the line-type light emitted from the light output part 140 is connected to the stage 130 and disposed above the glass substrate 120 inside the scanner body.

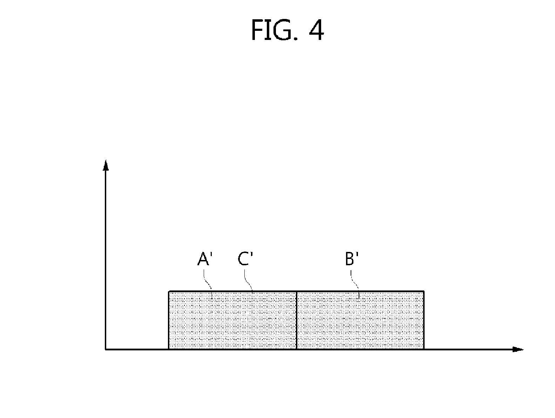

FIG. 4 is an intensity profile of the light output from the light source including a beam homogenizer according to an exemplary embodiment of the present invention.

A beam homogenizer can be additionally disposed between the output stage of the light source 210 (FIG. 2) and the optical system 220 (FIG. 2). When the beam homogenizer is used at the output stage of the light source, distribution of the line-type light output from the light output part is converted to stepwise profiles A' and B' having the uniform intensity, rather than the Gaussian profile.

Thus, when the location of the light output part is properly regulated in both sides of the glass substrate, it is possible to emit the line-type light having the uniform light intensity distribution C' across both sides of the glass substrate.

In so doing, in the stepwise; that is, quadrangular profile, the high quadrangular aspect ratio is determined by specification of the beam homogenizer to employ.

When the beam homogenizer of the high aspect ratio is used, the intensity of the line-type light reduces a little compared to FIG. 3 but the line-type light of the uniform intensity can be emitted to the both sides of the glass substrate more widely.

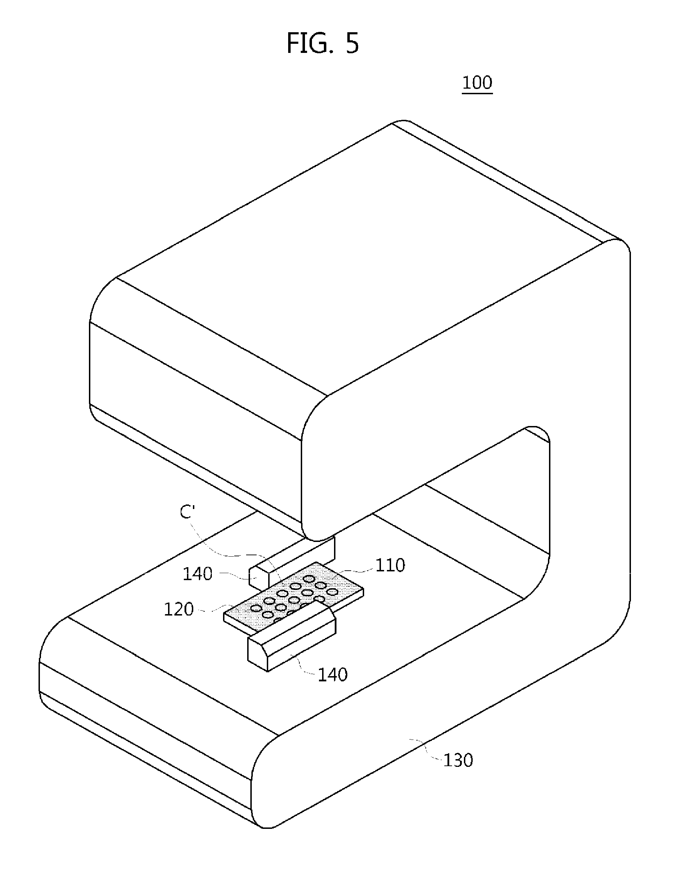

Hence, to make the uniform light intensity as in the nanoscale biochip scanner (FIG. 1) according to an exemplary embodiment of the present invention, one end of the light output part in one side of the substrate can be disposed relatively in parallel with the other end of the opposite light output part as shown in FIG. 5, without having to misalign the light output parts by the certain distance a.

By misaligning the light output pats for outputting the line-type light in both sides of the substrate, the nanoscale biochip scanner of the present invention can analyze a plurality of bio samples prepared in a certain region. In addition, the nanoscale biochip scanner can analyze a minimum bio sample by equalizing the intensity of the fluorescence.

Although the present disclosure has been described with an exemplary embodiment, various changes and modifications may be suggested to one skilled in the art. It is intended that the present disclosure encompass such changes and modifications as fall within the scope of the appended claims.

## Claims

1. A biochip scanner comprising:
a stage for holding a substrate on which a biochip is formed;
a plurality of light output parts formed on the stage for outputting a line-type light to both sides of the substrate; and
a camera disposed above the substrate.

2. The biochip scanner of claim 1, wherein the light output parts are misaligned in both sides of the substrate.

3. The biochip scanner of claim 1, wherein the light output part comprises:
a geometric optical system for producing a light emitted from a light source as a line light;
an optical splitter and a plurality of mirrors for splitting the line light to a plurality of paths; and
an light output part for emitting the line light split by the optical splitter, in both sides of the substrate.

4. The biochip scanner of claim 3, further comprising:
a beam homogenizer disposed at an output stage of the light source, for making uniform intensity distribution of the light.

5. The biochip scanner of claim 3, wherein one end of the light output part formed in one side of the substrate is formed in parallel with other end of the light output part formed in the other side of the substrate.

6. The biochip scanner of claim 3, wherein the light source is a laser oscillator or a laser diode.
